# DEMANDE DE BREVET EUROPEEN

(11) **EP 1 132 076 A1**
(43) Date de publication de la demande: **12.09.2001**
(21) Numéro de dépôt: 01400554.0
(22) Date de dépôt: 02.03.2001
(51) Int. Cl.: A61K 7/06

(54) **Composition capillaire épaissie comprenant un polymère fixant et un composé pulvérulent**

(30) Priorité: 07.03.2000 FR 0002907
(71) Demandeur: L'OREAL, 75008 Paris (FR)
(72) Inventeur: Belli, Emmanuelle, 92600 Asnieres (FR); Jourdan, Hervé, 78210 St-Cyr (FR); Pasquet, Dorothée, 92270 Bois-Colombes (FR)
(74) Mandataire: Bourdeau, Françoise

(57) **Abrégé**

L'invention concerne une composition cosmétique capillaire comprenant au moins un agent épaississant (a), un polymère fixant (b) et un composé pulvérulent (c), dans un milieu cosmétiquement acceptable, caractérisée par le fait que :
- l' agent épaississant (a) est choisi parmi les polymères naturels ou naturels modifiés (i), les copolymères réticulés d'acide acrylique et/ou méthacrylique (ii), les polyacrylamides épaississants réticulés (iii) et les polymères associatifs comportant au moins un motif hydrophile et au moins une chaîne grasse (iv) ;
- le polymère fixant (b) est un polymère non réticulé, et
- le composé pulvérulent (c) est formé par un matériau insoluble non lamellaire.

## Description

L'invention a pour objet une composition cosmétique capillaire, notamment un gel, comprenant au moins un polymère épaississant, un polymère fixant et un composé pulvérulent insoluble. Par « insoluble », on entend que le composé présente une solubilité dans la composition inférieure à 0,1 % en poids, la solubilité représentant la quantité de composé capable de se dissoudre dans 100 grammes de la composition.

L'invention vise également un procédé cosmétique capillaire comprenant l'application de cette composition sur les cheveux ainsi que son utilisation pour fixer et/ou maintenir la coiffure.

Au sens de la présente invention, on entend par « gel », une composition -initialement liquide, épaissie grâce à un agent ayant des propriétés épaississantes et/ou gélifiantes, ayant in fine une viscosité minimale de 160 cps, de préférence 250 cps à 25°C (viscosimètre Rhéomat 180 - mobile 2 - lecture après 30s).

Il est connu des produits de fixation et/ou de maintien de la coiffure se présentant sous diverses formes, notamment sous forme de gels. Si ceux-ci permettent souvent la fixation durable de la coiffure, ils présentent néanmoins divers inconvénients.

En particulier, de nombreux consommateurs reprochent que le gel reste collé sur les mains lors de l'application, que son temps de séchage sur les cheveux est long, ce qui peut entraîner un effondrement de la coiffure si le gel est appliqué en finition sur cheveux secs, et qu'il donne aux cheveux un aspect artificiel, en particulier une brillance non naturelle.

Pour résoudre les problèmes ci-dessus, il a déjà été proposé de réaliser des produits cosmétiques capillaires comprenant un polymère fixant, un agent épaississant et un agent pulvérulent. Néanmoins, les compositions connues à ce jour comprenant ces constituants ne donnent pas encore totalement satisfaction, dans la mesure où la coiffure manque encore de naturel et où les gels continuent à trop coller sur les mains lors de l'application et à sécher trop lentement.

Le problème posé par l'invention est de fournir une composition cosmétique capillaire, notamment sous forme de gel, qui soit améliorée par rapport aux compositions de l'art antérieur et, en particulier, qui laisse les mains propres après application sur les cheveux, donne lieu à une application sur des cheveux secs sans déstructuration la coiffure, sèche rapidement, maintienne fortement la forme de la coiffure et se répartisse de manière homogène.

De manière surprenante et inattendue, la Demanderesse a découvert qu'en sélectionnant judicieusement le polymère fixant, l'agent épaississant et l'agent pulvérulent pour réaliser la composition cosmétique capillaire, il est possible de résoudre le problème posé ci-dessus.

L'invention a pour objet une composition cosmétique capillaire comprenant au moins un agent épaississant (a), un polymère fixant (b) et un composé pulvérulent (c), dans un milieu cosmétiquement acceptable, caractérisée par le fait que :
- l'agent épaississant (a) est choisi parmi les polymères naturels ou naturels modifiés (i), les copolymères réticulés d'acide acrylique et/ou méthacrylique (ii), les polyacrylamides épaississants réticulés (iii) et les polymères associatifs comportant au moins un motif hydrophile et au moins une chaîne grasse (iv) ;
- le polymère fixant (b) est un polymère non réticulé, et
- le composé pulvérulent (c) est formé par un matériau insoluble non lamellaire.

Un autre objet de l'invention concerne un procédé cosmétique capillaire, en particulier un procédé de fixation et/ou de maintien de la coiffure mettant en oeuvre ladite composition.

Un autre objet de l'invention concerne l'utilisation de cette composition pour le maintien et/ou à la mise en forme de la coiffure.

Par épaississant naturel modifié on entend selon l'invention tout polymère épaississant obtenu par modification chimique simple à partir du polymère naturel lui-même.

On peut citer comme épaississant naturels ou naturels modifiés (i) convenant pour l'invention les gommes de xanthane, de scléroglucane, de gellane, de rhamsan, les alginates, la maltodextrine, l'amidon et ses dérivés, la gomme de karaya, la farine de caroube, les gommes de guar modifiées ou non et leurs dérivés, comme hydroxypropylguar, les celluloses et leurs dérivés. On utilise notamment les celluloses épaississantes et leurs dérivés (hydroxyéthycellulose, hydroxypropylcellulose, carboxyméthylcellulose), la gomme de guar et ses dérivés ( hydroxypropylguar), les gommes d'origine naturelle microbienne (gomme de xanthane, gomme de scléroglucane).

Les copolymères d'acide acrylique et/ou méthacrylique réticulés (ii) sont notamment les copolymères acide acrylique/acrylate d'éthyle et les polymères carboxyvinyliques. De tels polymères sont notamment les "carbomer" (CTFA) vendus par la société GOODRICH sous la dénomination Carbopol.

Les polyacrylamides épaississants réticulés (iii) peuvent plus particulièrement être choisis parmi :
- les homopolymères de 2-acrylamido-2-méthylpropane sulfonique réticulés,
- les copolymères d'acrylamide et d'acrylate d'ammonium éventuellement réticulés,
- les copolymères d'acrylamide (ou de méthacrylamide) et de chlorure de méthacryloyloxyéthyl triméthylammonium réticulés,
- les copolymères d'acrylamide et d'acide 2-acrylamido 2-méthyl propanesulfonique partiellement ou totalement neutralisés réticulés.

Comme copolymères réticulés d'acrylamide / acrylate d'ammonium, utilisés conformément à la présente invention, on peut plus particulièrement citer les copolymères acrylamide / acrylate d'ammonium (5/95 en poids) réticulé par un agent de réticulation à polyinsaturation oléfinique, tel que le divinylbenzène, le tétraallyloxyéthane, le méthylène bis-acrylamide, l'éther diallylique, des éthers polyallylpolyglycéryliques ou les éthers allyliques d'alcool de la série des sucres, tels que l'érythritol, le pentaérythritol, l'arabitol, le mannitol, le sorbitol ou le glucose.

Des copolymères analogues sont décrits et préparés dans le brevet français FR-2.416.723 et les brevets US-2.798.053 et US-2.923.692.

On utilise en particulier ce type de copolymère réticulé sous forme d'émulsion eau-dans-huile constituée d'environ 30 % en poids dudit copolymère, 25 % en poids d'huile de paraffine, 4 % en poids de mélange de stéarate de sorbitan et d'un dérivé éthoxylé hydrophile et 41 % en poids d'eau. Une telle émulsion est commercialisée sous la dénomination "BOZEPOL C" par la Société HOECHST.

Les copolymères d'acrylamide et de l'acide 2-acrylamido 2-méthyl propane sulfonique, utilisés conformément à la présente invention, sont des copolymères réticulés par un composé à polyinsaturation oléfinique, tels que ceux évoqués précédemment, et partiellement ou totalement neutralisés par un agent de neutralisation tel que la soude, la potasse, l'ammoniaque ou une amine telle que la triéthanolamine ou la monoéthanolamine.

Ils peuvent être préparés en copolymérisant l'acrylamide et le 2-acrylamido 2-méthylpropane sulfonate de sodium par voie radicalaire au moyen d'agents initiateurs du type azobisisobutyronitrile et par précipitation dans un alcool tel que le tertiobutanol.

On utilise plus particulièrement des copolymères obtenus par copolymérisation de 70 à 55 % en moles d'acrylamide et de 30 à 45 % en moles de 2-acrylamido 2-méthylpropane sulfonate de sodium. L'agent de réticulation étant utilisé à des concentrations de 10⁻⁴ à 4.10⁻⁴ mole par mole du mélange de monomères.

Ces copolymères particuliers sont incorporés dans les compositions de l'invention, de façon préférentielle, sous forme d'émulsions eau dans l'huile contenant de 35 à 40 % en poids de ce copolymère, de 15 à 25 % en poids d'un mélange d'hydrocarbures isoparaffiniques en C₁₂-C₁₃, de 3 à 8 % en poids de lauryléther de polyéthylèneglycol à 7 moles d'oxyde d'éthylène et d'eau. Une telle émulsion est commercialisée sous le nom de "SEPIGEL 305" par la société SEPPIC.

Le copolymère d'acrylamide et de chlorure de méthacryloyl oxyéthyl triméthylammonium réticulé, utilisé selon l'invention, est plus particulièrement un copolymère obtenu par copolymérisation de l'acrylamide et du diméthylaminoéthylméthacrylate quaternisé par le chlorure de méthyle, suivie d'une réticulation par un composé à insaturation oléfinique, en particulier le méthylène-bis acrylamide.

On utilise plus particulièrement un copolymère réticulé acrylamide/chlorure de méthacryloyl oxyéthyl triméthylammonium (environ 50/50 en poids) sous forme de dispersion contenant 50 % en poids dudit copolymère dans de l'huile minérale. Cette dispersion est commercialisée sous le nom de "SALCARE SC92" par la Société ALLIED COLLOIDS.

Les copolymères non réticulés de méthacrylamide et de chlorure de méthacryloyloxyéthyl triméthylammonium sont par exemple les produits vendus sous les dénominations commerciales ROHAGIT KF 400 et KF720 par la société ROHM et Haas.

Les polymères associatifs (iv) de l'invention peuvent appartenir à la classe des polyuréthannes.

Les polyuréthannes associatifs sont des copolymères séquencés comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

En particulier, ces polymères comportent au moins deux chaînes lipophiles hydrocarbonées, ayant de C₆ à C₃₀ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polymères peuvent être séquencés sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé par exemple). Les polymères peuvent être également en greffons ou en étoile.

De préférence, les polymères sont des copblymères triblocs dont la séquence hydrophile est une chaîne polyoxyéthylénée comportant de 50 à 1 000 groupements oxyéthylénés. En général les polyuréthannes associatifs comportent une liaison uréthanne entre les séquences hydrophiles, d'où l'origine du nom.

Par extension figurent aussi parmi les polyuréthannes associatifs, des polymères dont les séquence hydrophiles sont liées par d'autres liaisons chimiques aux séquences lipophiles.

A titre d'exemple, des polymères associatifs utilisables dans l'invention, on peut citer le polymère C₁₆-OE₁₂₀-C₁₆ vendu par la société HULS (sous le nom Sérad FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 ou 204. Ces polyuréthannes associatifs sont vendus sous forme pure.

Le produit DW 1206B de chez RHOM & HAAS à chaîne alkyle en C₂₀ et à liaison uréthanne, vendu à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Sérad FX1010 et le Sérad 1035 vendus par la société HULS, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS.

Les polymères utilisables dans l'invention sont en particulier ceux décrits dans l'article de G. Fonnum, J. Bakke et Fk. Hansen - Colloid Polym. Sci 271, 380.389 (1993).

Les polyuréthannes associatifs (iv) peuvent particulièrement être choisis parmi :
- les polyéthers polyuréthanes (a) susceptibles d'être obtenus par polycondensation d'au moins trois composés comprenant (i) au moins un polyéthylèneglycol comprenant de 50 à 500 moles d'oxyde d'éthylène, (ii) au moins un alcool gras en C₈-C₃₀ et (iii) au moins un diisocyanate et,
- les polyéthers polyuréthanes (b) susceptible d'être obtenus par polycondensation d'au moins trois composés comprenant (i) au moins un polyéthylèneglycol comprenant de 50 à 500 moles d'oxyde d'éthylène, (ii) au moins un alcool gras en C₈-C₃₀ différant de celui du polyéther polyuréthane (a) et (iii) au moins un diisocyanate.

De façon préférentielle, on utilisera des polyéthers polyuréthanes (a) et (b) pour lesquels le polyéthylène glycol comporte 150 ou 180 moles d'oxyde d'éthylène.

Toujours de façon préférentielle, on utilisera des polyéthers polyuréthanes (a) et (b) pour lesquels le diisocyanate est le méthylène bis(4-cyclohexylisocyanate).

Une composition plus particulièrement préférée selon l'invention est une composition telle que définie ci-dessus, pour laquelle, le polyéther polyuréthane (a) est obtenu par polycondensation d'au moins trois composés comprenant, un polyéthylèneglycol à 150 ou 180 moles d'oxyde d'éthylène, l'alcool stéarylique (C18) et le méthylène bis(4-cyclohexylisocyanate), et le polyéther polyuréthane (b) est obtenu par polycondensation d'au moins trois composés comprenant, un polyéthylèneglycol à 150 ou 180 moles d'oxyde d'éthylène, l'alcool décylique (C10) et le méthylène bis(4-cyclohexylisocyanate).

Parmi les polyéthers polyuréthanes (a), on peut citer le produit vendu par la société ROHM & HAAS sous la dénomination commerciale :
ACULYN 46, qui est un polycondensat comprenant au moins comme éléments, un polyéthylèneglycol à 150 du 180 moles d'oxyde d'éthylène, de l'alcool stéarylique et du méthylène bis(4-cyclohexyl-isocyanate) (SMDI), à 15% en poids dans une matrice de maltodextrine (4%) et d'eau (81%).

Parmi les polyéthers polyuréthanes (b), on peut citer le produit vendu par la société ROHM & HAAS sous la dénomination commerciale :
ACULYN 44, qui est un polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propylèneglycol (39%) et d'eau (26%).

On peut également utiliser, en tant que polyuréthanne associatif, le Viscophobe DB 1000 (Union Carbide).

Comme autres polymères associatifs utilisables, on peut notamment citer les copolymères acide acrylique (Meth) / acrylate d'alkyle C10-C30 comme le Pemulen TR1 de Goodrich, les celluloses -non-ioniques à chaîne grasse comme le Natrosol Plus Grade 330 CS de Aqualon, les celluloses cationiques à chaîne grasse comme le CRODACEL QS de chez CRODA.

Le polymère fixant non réticulé (b) est généralement choisi parmi les polymères fixants anionique, cationique, amphotère, non ionique et leurs mélanges. Un polymère fixant est un polymère capable de maintenir et/ou de fixer la forme de la coiffure.

Ces polymères fixants peuvent être utilisés sous forme solubilisée ou encore sous forme de dispersion de particules solides de polymère.

Les polymères fixants (b) cationiques, anioniques, amphotères et non ioniques utilisables conformément à l'invention sont décrits ci-après.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 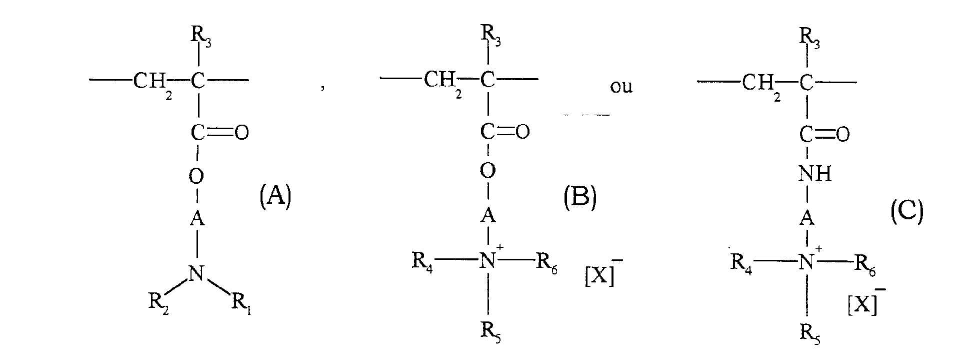 dans lesquelles:
   R₃ désigne un atome d'hydrogène ou un radical CH₃;
   A est un groupe alkyle linéaire ou ramifié de 1 à 6 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle;
   R₁ et R₂ représentent hydrogène ou un groupe alkyle ayant de 1 à 6 atomes de carbone;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs, des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyl-triméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxy-éthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - et le copolymère vinylpyrrolidone / méthacrylamide de diméthylaminopropyle quaternisé tel que le produit vendu sous la dénomination "GAFQUAT HS 100" par la société ISP.
(2) les polysaccharides quaternisés décrits plus particulièrement dans les brevets américains 3.589.578 et 4.031.307 tel que les gommes de guar contenant des groupements cationiques trialkylammonium.
   De tels produits sont commercialisés notamment sous les dénominations commerciales de JAGUAR C13 S, JAGUAR C 15, JAGUAR C 17 par la société MEYHALL.
(3) les copolymères quaternaires de vinylpyrrolidone et de vinylimidazole tels que les produits commercialisés par BASF sous l'appellation Luviquat TFC;
(4) les chitosanes ou leurs sels; les sels utilisables sont en particulier les acétate, lactate, glutamate, gluconate ou le pyrrolidone carboxylate de chitosane.
   Parmi ces composés, on peut citer le chitosane ayant un taux de désacétylation de 90,5% en poids vendu sous la dénomination KYTAN BRUT STANDARD par la société ABER TECHNOLOGIES, le pyrrolidone carboxylate de chitosane vendu sous la dénomination KYTAMER PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants anioniques généralement utilisés sont des polymères comportant des groupements dérivés d'acide carboxylique, sulfonique ou phosphorique et ont un poids moléculaire compris entre environ 500 et 5.000.000.

1) Les groupements carboxyliques sont apportés par des monomères mono ou diacides carboxyliques insaturés tels que ceux répondant à la formule : 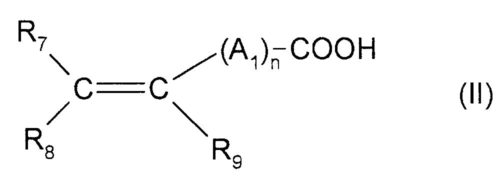 dans laquelle n est un nombre entier de 0 à 10, A₁ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;

Dans la formule précitée un radical alkyle inférieur désigne de préférence un groupement ayant 1 à 4 atomes de carbone et en particulier, méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL E ou K par la société ALLIED COLLOID et ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀ par exemple de lauryle tel que celui vendu par la société ISP sous la dénomination ACRYLIDONE LM et les terpolymères acide méthacrylique/ acrylate d'éthyle/ acrylate de tertiobutyle tel que le produit vendu sous la dénomination LUVIMER 100 P par la société BASF.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α-ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
D) les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. ; De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
   - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
   - les fonctions anhydrides de ces copolymères étant éventuellement monoestérifiées ou monoamidifiées.

Ces polymères sont par exemple décrits dans les brevets français 2.350.384 et 2.357.241 de la demanderesse.

E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères fixants anioniques sont de préférence choisis parmi les copolymères d'acide acrylique tels que le terpolymère acide-acrylique / acrylate d'éthyle / N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX ou MAE par la société BASF, le copolymère acétate de vinyle/acide crotonique vendu sous la dénomination LUVISET CA 66 par la société BASF et le copolymère acétate de vinyle/acide crotonique greffé par du polyéthylèneglycol sous la dénomination ARISTOFLEX A par la société BASF.

Les polymères fixants anioniques les plus particulièrement préférés sont choisis parmi le copolymère méthylvinyléther / anhydride maléique mono estérifié vendu sous la dénomination GANTREZ ES 425 par la société ISP, le terpolymère acide acrylique / acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle */* tertio-butyl benzoate de vinyle */* acide crotonique et les terpolymères acide crotonique / acétate de vinyle / néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX OU MAE par la société BASF, le terpolymère de vinylpyrrolidone / acide acrylique/méthacrylate de lauryle vendu sous la dénomination ACRYLIDONE LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes;
B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants:

1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylamino-alkylméthacrylamide et acrylamide. De tels composés sont décrits dans le brevet américain n° 3 836 537.

(2) les polymères comportant des motifs dérivant :
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
Les comonomères basiques préférés sont des méthacrylates d'amino-éthyle, de butyl aminoéthyle, de N,N'-diméthylaminoéthyle, de N-tertio-butyl-aminoéthyle.
On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.

(3) les polyamino amides réticulés et alcoylés partiellement ou totalement dérivant de polyaminoamides de formule générale III: 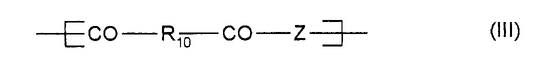 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
a) dans les proportions de 60 à 100 moles %, le radical IV 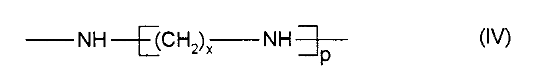 où x=2 et p=2 ou 3, ou bien x=3 et p=2
   ce radical dérivant de la diéthylène triamine, de la triéthylène tétraamine ou de la dipropylène triamine;
b) dans les proportions de 0 à 40 moles % le radical (IV) ci-dessus, dans lequel x=2 et p=1 et qui dérive de l'éthylènediamine, ou le radical dérivant de la pipérazine : 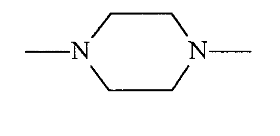
c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.

(4) les polymères comportant des motifs zwittérioniques de formule V: 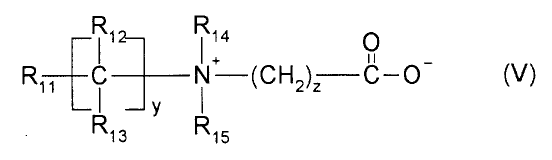 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.

Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.

A titre d'exemple, on peut citer le copolymère de méthacrylate de méthyle / diméthyl carboxyméthylammonio éthylméthacrylate de méthyle tel que le produit vendu sous la dénomination DIAFORMER Z301 par la société SANDOZ.

(5) les polymères dérivés du chitosane comportant des motifs monomères répondant aux formules suivantes : 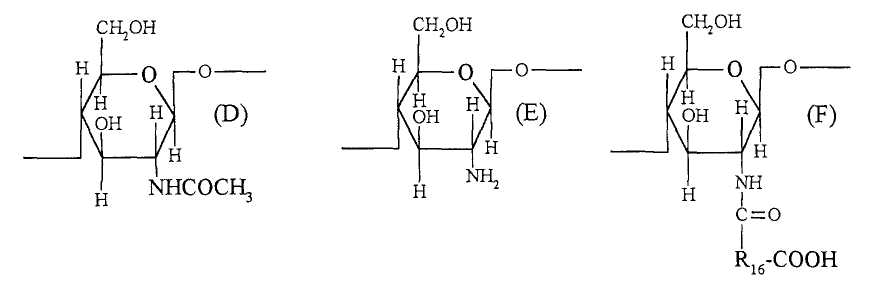 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 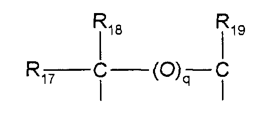 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcolylthio, sulfonique, un reste alcoylthio dont le groupe alcoyle porte un reste amino, l'un au moins des radicaux R₁₇, R₁₈ et R₁₉ étant dans ce cas un atome d'hydrogène ;
ou si q=1, R₁₇, R₁₈ et R₁₉ représentent chacun un atome d'hydrogène, ainsi que les sels formés par ces composés avec des bases ou des acides.

(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.

(7) Les polymères répondant à la formule générale (VI) par exemple décrits dans le brevet français 1 400 366 : 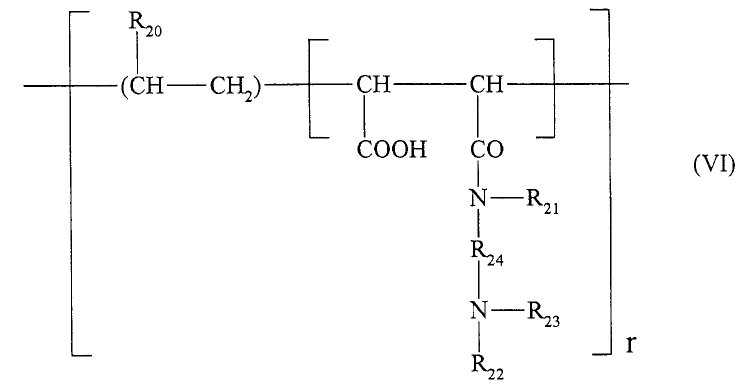 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus,
ainsi que les homologues supérieurs de ces radicaux et contenant jusqu'à 6 atomes de carbone.

(8) Des polymères amphotères du type -D-X-D-X- choisis parmi:
a) les polymères obtenus par action de l'acide chloracétique ou le chloracétate de sodium sur les composés comportant au moins un motif de formule :

   -D-X-D-X-D- (VII)

   où D désigne un radical 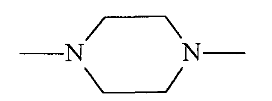 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
b) Les polymères de formule :

   -D-X-D-X- (VII')

   où D désigne un radical 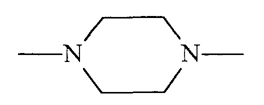 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) les copolymères alkyl(C1-C5)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/butylaminoethylmethacrylate copolymer tels que les produits vendus sous les dénominations AMPHOMER, AMHOMER LV 71 ou LOVOCRYL 47 par la société NATIONAL STARCH et ceux de la famille (4) tels que les copolymère de méthacrylate de méthyle / diméthyl carboxyméthylammonio éthylméthacrylate de méthyle par exemple vendu sous la dénomination DIAFORMER Z301 par la société SANDOZ.

Les polymères fixants non ioniques utilisables selon la présente invention sont choisis par exemple parmi :
- les homopolymères de vinylpyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.

Les radicaux alkyle des polymères non ioniques ont de 1 à 6 atomes de carbone sauf mention contraire.

Les polymères non ioniques qui conviennent tout particulièrement pour la réalisation des compositions conformes à l'invention sont ceux choisis parmi:
* les copolymères de vinyllactame tels que les copolymères de vinylpyrrolidone et d'acétate de vinyle et les copolymères vinylpyrrolidone/ acétate de vinyle/ propionate de vinyle
* la polyvinylcaprolactame LUVISKOL PLUS (BASF)
* les homopolymères d'acétate de vinyle tels que APPRETAN EM (HOECHST) ou RHODOPAS A 012 (RHONE POULENC)
* les polyalkyloxazolines tels que PEOX 50 000 et PEOX 500 000 (DOW CHEMICAL)
* les copolymères d'acétate de vinyle et d'ester acrylique tels que le RHODOPAS AD 310 (RHONE POULENC)
* les copolymères d'acétate de vinyle et d'éthylène tels que APPRETAN TV (HOECHST)
* les copolymères d'acétate de vinyle et d'ester maléique tels que APPRETAN MB EXTRA (HOECHST)
* les homopolymères d'acrylates d'alkyle et les homopolymères de métacrylates d'alkyle tels que LUHYDRAN A 848 S (BASF)
* les copolymères d'esters acryliques tels que PRIMAL AC-261 K (ROHM & HAAS), ACRONAL 601 (BASF) ou APPRETAN N 9213 (HOECHST)
* les copolymères d'acrylonitrile et d'un monomère non-ionique tels que CJ 0601 B (ROHM & HAAS)
* les polyuréthanes tels que ACRYSOL RM 1020 ou ACRYSOL RM 2020 (ROHM & HAAS)
* les copolymères d'acrylate d'alkyle et d'uréthane tels que 8538-33 (NATIONAL STARCH)
* les polyamides tels que ESTAPOR LO 11 (RHONE POULENC)

Selon l'invention, on peut également utiliser les polymères fixants de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule: 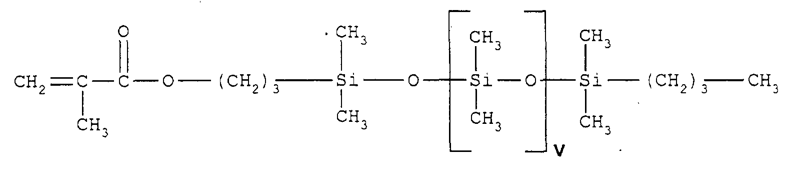 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut aussi utiliser, comme polymères fixants, des polyuréthannes fonctionnalisés ou non, siliconés ou non.

Les polyuréthannes particulièrement visés par la présente invention sont ceux décrits dans les brevets EP 0 751 162, EP 0 637 600, FR 2 743 297 et EP 0 648 485 dont la Demanderesse est Titulaire, ainsi que le brevets EP 0 656 021 ou WO 94/03510 de la Société BASF et EP 0 619 111 de la Société National Starch.

Les composés pulvérulents non lamellaires insolubles (c) peuvent être choisis parmi les produits suivants, étant entendu que si plusieurs formes sont possibles, seules les formes non lamellaires font partie de l'invention :
- le talc,
- le kaolin, qui est un silicate d'aluminium hydraté,
- les poudres de Nylon par exemple celle commercialisée sous la dénomination "ORGASOL 2002 ED NAT COS" par la Société ATOCHEM,
- les poudres de polyéthylène, par exemple celle commercialisée sous la dénomination "COATHYLENE HA 1681" par la Société PLAST LABOR,
- les poudres de poly-β-alanine,
- les poudres polyfluorées notamment de polytétrafluoroéthylène, par exemple celle commercialisée sous la dénomination "MP 1400" par la Société DUPONT DE NEMOURS,
- les poudres de copolymère acrylique, telles que celles commercialisées sous la dénomination "POLYTRAP Q5 6603" par la Société DOW CHEMICA,
- les poudres de polystyrène telles que celles commercialisées sous la dénomination "POLYSPHERE 3 000 SP" par la Société PRESPERESE
- les poudres de polyester,
- les microsphères expansées en matériau thermoplastique, par exemple celle commercialisée sous la dénomination "EXPANCEL 551 DE" par la Société EXPANCEL,
- les microbilles de résine de silicone (Tospearls de la Société TOSHIBA, par exemple),
- les oxydes de zinc et de titane,
- les oxydes d'aluminium, de zirconium ou de cérium,
- le carbonate de calcium précipité, le carbonate et l'hydrocarbonate de magnésium,
- l'hydroxyapatite,
- les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium,
- les silices,
- les poudres de polymères hydrophiles, qui sont d'origine synthétique comme les polyacrylates, par exemple celui commercialisé sous la dénomination "MICROPEARL M 100" par la Société MATSUMOTO,
- les polyamides acryliques tels que ceux commercialisés par la Société ORIS,
- les polyuréthannes insolubles tels que celui commercialisé sous la dénomination "PLASTIC POWDER D 800" par la Société TOSHNU
- les microsphères poreuses de cellulose.

Les composés pulvérulents peuvent également être des composés qui ont été rendus hydrophiles par enrobage ou greffage chimique à l'aide de matières telles que le chitosane, le dioxyde de titane, la silice ou des polymères hydrophiles, notamment des polyesters sulfoniques ou des polyammonium quaternaire.

A titre d'exemple, on peut citer le talc enrobé de chitosane vendu par Daito sous le nom « Talc CT 2 MSA ».

On peut également choisir des composés pulvérulents hydrophobes, choisis parmi des composés pulvérulents de nature aussi bien hydrophobe qu'hydrophile. Dans ce dernier cas, ils sont rendus hydrophobes par enrobage ou greffage chimique par des produits tels que les silicones, les aminoacides, les savons métalliques, les dérivés fluorés, les huiles minérales, la lécithine, le triisostéaroyl titanate d'isopropyle, le polyéthylène, le collagène et ses dérivés, les polyacrylates.

A titre d'exemple, on peut citer les microbilles de silice enrobées de polymethylhydrogenosiloxane vendues sous la dénomination commerciale "Silice SI SB 700" par Miyoshi ou encore la sericite enrobée de methicone/huile d'oeuf hydrogénée vendue sous la dénomination commerciale "Sericite SNI S100" par Miyoshi.

De préférence, la taille des particules du matériau pulvérulent est comprise entre 10 nm et 100 µ.

La « taille des particules » est la distance maximale qu'il est possible de mesurer entre deux points diamétralement opposés de la particule. Cette taille est mesurée par exemple au moyen de granulomètre laser.

Avantageusement, l'agent épaississant (a) est présent dans la composition conforme à l'invention à une concentration relative en poids comprise entre 0,01 et 5 %, et de préférence comprise entre 0,1 et 2 %.

Avantageusement, le polymère fixant non réticulé (b) est présent dans la composition conforme à l'invention à une concentration relative en poids comprise entre 0,1 et 10 %, de préférence comprise entre 0,5 et 5 %.

Avantageusement, le matériau pulvérulent (c) est présent dans la composition conforme à l'invention à une concentration relative en poids comprise entre 0,1 et 10 %, et de préférence comprise entre 1 et 5 %.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄.

Parmi ces alcools, on peut citer l'éthanol, l'isopropanol. L'éthanol est particulièrement préféré.

La composition de l'invention peut également contenir au moins un additif choisi parmi les tensioactifs anioniques, cationiques, non ioniques ou amphotères autres que ceux de l'invention, les parfums, les filtres, les conservateurs, les protéines, les vitamines, les provitamines, les polymères, les huiles végétales, minérales ou synthétiques, les polyols comme les glycols ou la glycérine, les silicones, les alcools gras et tout autre additif classiquement utilisé dans les compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient'pas ou substantiellement pas, altérées par l'addition envisagée.

Les compositions conformes à l'inventions peuvent être appliquées sur la peau, les ongles, les lèvres, les cheveux, les sourcils et les cils.

Les compositions conformes à l'invention sont particulièrement adaptées pour des cheveux secs ou humides, en tant que produits de coiffage.

L'invention va être plus complètement illustrée à l'aide des exemples non limitatifs suivants.

Tous les pourcentages sont des pourcentages relatifs en poids par rapport au poids total de la composition et m.a. signifie matière active.

Les constituants utilisés sont rassemblés dans le tableau ci-après.

| | |
|---|---|
| Synthalen K | Homopolymère d'acide acrylique réticulé commercialisé par 3V |
| Gafquat | Polyquaternium 11 commercialisé par ISP |
| Klucel EF | Hydroxypropylcellulose commercialisé par Aqualon |
| Copolymère 845 | PVP/diméthylaminométhacrylate commercialisé par ISP |
| PVP | Polyvinylpyrrolidone |
| VA | Vinyl acétate |
| Mirasil | Diméthicone copolyol commercialisé par Rhône Poulenc |
| Orgasol 2002 | Nylon 12 commercialisé par Elf Atochem |
| Expancel 551 DE | Microsphères vinyliques expancées par de l'isobutane commercialisées par Expancel |
| Jaguar HP 105 | Gomme commercialisée par Rhône Poulenc |
| Kytamer | Chitosan glycolate commercialisé par Amerhold |
| Acumist B6 | Poudre de polyéthylène commercialisée par Allied |
| Simulsol 165 | PEG200 Glycéryl stéarate commercialisé par Seppic |
| Brij 78 | Alcool alcoxylé commercialisé par Uniquema |
| Carbopol Ultrez 10 | Homopolymère d'acide acrylique réticulé commercialisé par Goodrich |
| Procétyl AWS | Alcool alcoxylé commercialisé par Croda |
| Belsil DMC 6038 | Diméthicone copolyol commercialisé par Wacker Chemie |

### EXEMPLES :

On réalise divers gels conformes à l'invention.

### Exemple 1

On réalise les gels A à E conformes à l'invention.

| | **A** | **B** | **C** | **D** | **E** |
|---|---|---|---|---|---|
| Synthalen K | 0.5 | | | | |
| Gafquat 755 | 1 | | | | |
| Klucel EF | 0.25 | | | | |
| Copolymère 845 | 1.5 | | | | |
| PVP | 3 | | | | |
| PVP/VA | | | | 3 | |
| Mirasil DMCO (Rhodia) | 0.15 | | | | |
| Kaolin | 3 | | | | |
| Orgasol 2002 | | 5 | | | |
| MSX 4562 (microsphères silice/alumine) | | | 10 | | |
| Expancel 551 DE | | | | 1 | |
| Talc | | | | | 5 |
| Ethanol | 17.2 | | | | |
| Triéthanolamine | qs pH 7.5 | | | | |
| Eau | qsp 100 | | | | |

### Exemple 2

On réalise les gels F et G conformes à l'invention.

| | **F** | **G** |
|---|---|---|
| Jaguar HP 105 | 1.3 | |
| DC Q2-5220 (Dow Corning) | 2 | |
| Kytamer PC (Amerchol) | 0.3 | |
| Orgasol 2002 | 5 | |
| Acumist B6 (poudre de PET) | | 5 |
| Ethanol | 17.2 | |
| Acide citrique | qs pH 4 | |
| Eau | qsp 100 | |

### Exemple 3

On réalise les gels H et I conformes à l'invention.

| | | **H** | **I** |
|---|---|---|---|
| Phase a | Alcool cétyl stéarylique 50/50 | 6 | |
| | Simulsol 165 (Seppic) | 6 | |
| | Brij 78 (Uniquema) | 10 | |
| | Conservateur | 0.3 | |
| Phase b | Carbopol Ultrez 10 | 0.3 | |
| | Amino méthyl propanol | qs pH 7.5 | |
| | PVP | 2 | |
| | Butylène glycol | 3 | |
| | Procetyl AWS (Croda) | 3 | |
| | Conservateur | 0.15 | |
| | Eau | qsp 100 | |
| Phase c | Kaolin | 1 | 3 |

Le mode opératoire est le suivant :
- Dans une cuve de fabrication, on prépare la phase b. Après épaississement du Carbopol dans l'eau, on neutralise à pH 7.5, on ajoute des autres matières premières et on chauffe à 80°C ;
- On réalise par ailleurs la phase a, par chauffage et solubilisation des matières premières à 80°C ;
- On verse a dans b, puis on ajoute la phase c.

### Exemple 4

On réalise le gel J conforme à l'invention.

| | J |
|---|---|
| Carbopol Ultrez 10 | 0.4 |
| Viscophobe DB 1000 (Amerchol) | 1.5 |
| PVP/VA | 3 |
| Belsil DMC 6038 | 0.5 |
| Kaolin | 1 |
| Ethanol | 17.2 |
| Amino méthyl propanol | qs pH 7.5 |
| Eau | qsp 100 |

Les gels réalisés ne collent pas aux doigts, et ils donnent aux cheveux un aspect naturel, tout en fixant la coiffure.

## Revendications

1. Composition cosmétique capillaire comprenant au moins un agent épaississant (a), un polymère fixant (b) et un composé pulvérulent (c), dans un milieu cosmétiquement acceptable, **caractérisée par** le fait que :
- l' agent épaississant (a) est choisi parmi les polymères naturels ou naturels modifiés (i), les copolymères réticulés d'acide acrylique et/ou méthacrylique (ii), les polyacrylamides épaississants réticulés (iii) et les polymères associatifs comportant au moins un motif hydrophile et au moins une chaîne grasse (iv) ;
- le polymère fixant (b) est un polymère non réticulé, et
- le composé pulvérulent (c) est formé par un matériau insoluble non lamellaire.

2. Composition selon la revendication 1, **caractérisée par** le fait que le polymère naturel ou naturel modifié (i) est choisi parmi les gommes de xanthane, de scléroglucane, de gellane, de rhamsan, les alginates, la maltodextrine, l'amidon et ses dérivés, la gomme de karaya, la farine de caroube, les gommes de guar et leurs dérivés, les celluloses épaississantes et leurs dérivés.

3. Composition selon la revendication 1, **caractérisée par** le fait que les copolymères d'acide acrylique et/ou méthacrylique réticulés (ii) sont choisis parmi les copolymères acide acrylique/acrylate d'éthyle et les polymères carboxyvinyliques.

4. Composition selon la revendication 1, **caractérisée par** le fait que les polyacrylamides épaississants réticulés (iii) sont choisis parmi :
- les homopolymères de 2-acrylamido-2-méthylpropane sulfonique réticulés,
- les copolymères d'acrylamide et d'acrylate d'ammonium éventuellement réticulés,
- les copolymères d'acrylamide (ou de méthacrylamide) et de chlorure de méthacryloyloxyéthyl triméthylammonium réticulés,
- les copolymères d'acrylamide et d'acide 2-acrylamido 2-méthyl propanesulfonique partiellement ou totalement neutralisés réticulés.

5. Composition selon la revendication 1, **caractérisée par** le fait que les polymères associatifs (iv) sont des polyuréthannes associatifs.

6. Composition selon l'une quelconque des revendications précédentes, **caractérisée par** le fait que le polymère fixant est choisi parmi les polymères fixants anionique, cationique, amphotère, non ionique et leurs mélanges.

7. Composition selon l'une quelconque des revendication précédentes,
**caractérisée par** le fait que le polymère fixant est un polymère anionique choisi parmi:
- les polymères comportant des motifs carboxyliques dérivant de monomères mono ou diacides carboxyliques insaturés de formule : 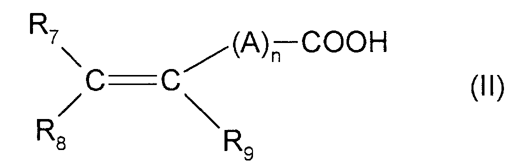 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;
- les polymères comprenant des motifs dérivant d'acide sulfonique tels que des motifs vinylsulfonique, styrènesulfonique, acrylamido alkylsulfonique.

8. Composition selon l'une quelconque des revendications 1 à 6, **caractérisée en ce que** le polymère fixant est un polymère amphotère choisi parmi les polymères comportant des motifs dérivant:
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

9. Composition selon l'une quelconque des revendications 1 à 6, **caractérisée par** le fait que le polymère fixant est un polymère non ionique choisi parmi :
- les polyalkyloxazolines;
- les homopolymères d'acétate de vinyle;
- les copolymères d'acétate de vinyle et d'ester acrylique;
- les copolymères d'acétate de vinyle et d'éthylène;
- les copolymères d'acétate de vinyle et d'ester maléïque;
- les copolymères de polyéthylène et d'anhydride maléïque;
- les homopolymères d'acrylates d'alkyle et les homopolymères de méthacrylates d'alkyle;
- les copolymères d'esters acryliques tels que par exemple les copolymères d'acrylates d'alkyle et de méthacrylates d'alkyle ;
- les copolymères d'acrylonitrile et d'un monomère non ionique choisi par exemple parmi le butadiène et les (méth)acrylates d'alkyle;
- les copolymères d'acrylate d'alkyle et d'uréthanne ; et
- les polyamides.

10. Composition selon la revendication 1 à 6, **caractérisée par** le fait que le polymère fixant est un polymère cationique choisi parmi :
- le copolymère d'acrylamide et de diméthylaminoéthyl méthacrylate quaternisé au sulfate de diméthyle,
- les copolymères d'acrylamide et de chlorure de méthacryloyloxyéthyltriméthylammonium,
- le copolymère d'acrylamide et de méthosulfate de méthacryloyloxyéthyltriméthylammonium,
- les copolymères vinylpyrrolidone / acrylate ou méthacrylate de dialkylaminoalkyle quaternisés ou non,
- les terpolymère méthacrylate de diméthyl amino éthyle/ vinylcaprolactame/ vinylpyrrolidone,
- le copolymère vinylpyrrolidone / méthacrylamide de diméthylaminopropyle quaternisé,
- les polysaccharides quaternisés, tels que les gommes de guar contenant des groupements cationiques trialkylammonium,
- les copolymères quaternaires de vinylpyrrolidone et de vinylimidazole,
- les chitosanes ou leurs sels,
- les dérivés de cellulose cationiques.

11. Composition selon l'une quelconque des revendications 1 à 6, **caractérisée par** le fait que le polymère fixant est un polyuréthanne fonctionnalisé ou non, siliconé ou non.

12. Composition selon l'une quelconque des revendications 1 à 6,
**caractérisée par** le fait que le polymère fixant est un polymère de type siliconé greffé comprenant une portion polysiloxane et une portion constituée d'une chaîne organique non-siliconée, l'une des deux portions constituant la chaîne principale du polymère l'autre étant greffée sur la dite chaîne principale.

13. Composition selon l'une quelconque des revendications précédentes, **caractérisée par** le fait que les composés pulvérulents sont choisis parmi les composés non lamellaires suivants :
- le talc
- le kaolin,
- les poudres de Nylon
- les poudres de polyéthylène
- les poudres de poly-β-alanine,
- les poudres polyfluorées,
- les poudres de copolymère acrylique,
- les poudres de polystyrène,
- les poudres de polyester,
- les microsphères expansées en matériau thermoplastique,
- les microbilles de résine de silicone,
- les oxydes de zinc et de titane,
- les oxydes d'aluminium, de zirconium ou de cérium,
- le carbonate de calcium précipité, le carbonate et l'hydrocarbonate de magnésium,
- l'hydroxyapatite,
- les savons métalliques dérivés d'acides organiques carboxyliques ayant de 8 à 22 atomes de carbone, de préférence de 12 à 18 atomes de carbone,
- les silices,
- les poudres de polymères hydrophiles, qui sont d'origine synthétique comme les polyacrylates,
- les polyamides acryliques,
- les polyuréthannes insolubles, et
- les microsphères poreuses de cellulose.

14. Composition selon l'une quelconque des revendications précédentes, **caractérisée par** le fait que l'agent épaississant (a) est présent dans la composition conforme à l'invention à une concentration relative en poids comprise entre 0,01 et 5 %, et de préférence comprise entre 0,1 et 2 %.

15. Composition selon l'une quelconque des revendications précédentes, **caractérisée par** le fait que le polymère fixant non réticulé (b) est présent dans la composition conforme à l'invention à une concentration relative en poids comprise entre 0,1 et 10 %, de préférence comprise entre 0,5 et 5 %.

16. Composition selon l'une quelconque des revendications précédentes, **caractérisée par** le fait que le matériau pulvérulent (c) est présent dans la composition conforme à l'invention à une concentration relative en poids comprise entre 0,1 et 10 %, et de préférence comprise entre 1 et 5 %.

17. Composition selon l'une quelconque des revendications précédentes, **caractérisée par** le fait que le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄.

18. Composition selon l'une quelconque des revendications précédentes, **caractérisée par** le fait qu'elle contient au moins un additif choisi parmi les tensioactifs anioniques, cationiques, non ioniques ou amphotères autres que ceux de l'invention, les parfums, les filtres, les conservateurs, les protéines, les vitamines, les provitamines, les polymères, les huiles végétales, minérales ou synthétiques, les polyols comme les glycols ou la glycérine, les silicones, les alcools gras et tout autre additif classiquement utilisé dans les compositions cosmétiques.

19. Procédé cosmétique capillaire, **caractérisé par** le fait qu'il comprend l'application d'une composition selon l'une quelconque des revendications précédentes.

20. Utilisation d'une composition selon l'une quelconque des revendications 1 à 18 pour fixer et/ou mettre en forme la coiffure.
